# EUROPEAN PATENT SPECIFICATION

(11) **EP 2 512 389 B1**
(45) Date of publication and mention of the grant of the patent: **02.09.2015**
(21) Application number: 10799173.9
(22) Date of filing: 14.12.2010
(51) Int. Cl.: A61F 9/00, A61F 9/007, A61K 9/00

(54) **INTRACAMERAL DEVICES FOR SUSTAINED DELIVERY**
INTRAKAMERALE VORRICHTUNGEN FÜR VERZÖGERTE FREISETZUNG
DISPOSITIFS INTRACAMÉRULAIRES POUR ADMINISTRATION PROLONGÉE

(30) Priority: 16.12.2009 US 287078 P
(43) Date of publication of application: 24.10.2012
(73) Proprietor: ALLERGAN, INC., Irvine, CA 92612 (US)
(72) Inventor: ROBINSON, Michael, R., Irvine California 92606 (US); WERHNER, Orilla, Anaheim California 92804 (US); NOVAKOVIC, Zoran, Irvine California 92614 (US); BURKE, James, Santa Ana California 92705 (US); GHEBREMESKEL, Alazar, Irvine California 92606 (US); SPADA, Lon, Walnut California 91789 (US)
(74) Representative: Hoffmann Eitle
(86) International application number: PCT/US2010/060314
(87) International publication number: WO 2011/075481

(56) References cited:
- WO-A1-2010/088548
- GB-A- 2 296 663
- US-A1- 2005 197 613
- US-A1- 2006 024 350
- US-A1- 2007 293 872
- US-A1- 2009 227 933

## Description

### CROSS REFERENCE

### FIELD OF THE INVENTION

The present invention relates to intracameral sustained release devices.

### BACKGROUND

Historically, treatment of eye conditions, or ocular conditions, has usually been effected through the use of applied, topical ophthalmic drugs in either liquid or ointment form. However, in many instances, it is preferable to release a pharmaceutical agent at a controlled and/or continuous rate over a prolonged period of time in order to obtain a desired pharmacological effect. It is well known that such continuous delivery of a drug, or a bioactive agent, is not obtainable through the use of liquid or ointment application, despite periodic application of these medications. Even with the controlled dispensing of liquid eye drops, for example, the level of medication in the eye varies dramatically because of the washing effect of tears which can substantially decrease the amount of available medication until the next application of drops.

As such, delivery of drugs or bioactive agents to different regions of the eye, such as the retina, vitreous, anterior chamber, sub-Tenon's space and uveal tract is typically achieved by high systemic dosing, intra-ocular injections or other measures. Penetration of systemically administered drugs into the retina or other portions of the eye is severely restricted by the blood-retinal barrier (BRB) for most compounds. Although intraocular injection, such as intravitreal injections, resolves some constraints posed by the BRB and significantly reduces the risk of systemic toxicity, intraocular injection techniques may result in retinal detachment, physical damage to the lens, exogenous endophthalmitis, and also may result in high pulsed concentrations of therapeutic agent at the lens and other intraocular tissues.

Another complication is that compounds are eliminated from the vitreous by diffusion to the retro-zonular space with clearance via the aqueous humor or by trans-retinal elimination. Most compounds utilize the former pathway while lipophilic compounds and those with trans-retinal transport mechanisms will utilize the latter. Unfortunately, compounds that are eliminated across the retina have extremely short half-lives. Hence, for these compounds it is difficult to maintain therapeutic concentrations by direct intraocular injection, and therefore, frequent injection is often required.

Even further, treating ocular conditions such as ocular hypertension, for example, by reducing or at least maintaining intraocular pressure, can be very challenging. Commonly, ocular hypertensive agents are used to treat such conditions. Ocular hypertensive agents, however, are useful in the treatment of a number of various ocular hypertensive conditions, such as post-surgical and post-laser trabeculectomy ocular hypertensive episodes, glaucoma, and as pre-surgical adjuncts.

Glaucoma is a disease of the eye characterized by increased intraocular pressure. On the basis of etiology, glaucoma has been classified as primary or secondary. For example, primary glaucoma in adults (congenital glaucoma) may be either open-angle or acute or chronic angle-closure. Secondary glaucoma results from pre-existing ocular diseases such as uveitis, intraocular tumor or an enlarged cataract.

The underlying causes of primary glaucoma are not yet known. The increased intraocular tension is due to the obstruction of aqueous humor outflow. In chronic open-angle glaucoma, the anterior chamber and its anatomic structures appear normal, but drainage of the aqueous humor is impeded. In acute or chronic angle-closure glaucoma, the anterior chamber is shallow, the filtration angle is narrowed, and the iris may obstruct the trabecular meshwork at the entrance of the canal of Schlemm. Dilation of the pupil may push the root of the iris forward against the angle, and may produce pupillary block and thus precipitate an acute attack. Eyes with narrow anterior chamber angles are predisposed to acute angle-closure glaucoma attacks of various degrees of severity and should be treated accordingly.

Secondary glaucoma is caused by any interference with the flow of aqueous humor from the posterior chamber of the eye into the anterior chamber and subsequently, into the canal of Schlemm. Inflammatory disease of the anterior segment may prevent aqueous escape by causing complete posterior synechia in iris bombe and may plug the drainage channel with exudates. Other common causes are intraocular tumors, enlarged cataracts, central retinal vein occlusion, trauma to the eye, operative procedures and intraocular hemorrhage.

Considering all types together, glaucoma occurs in about 2% of all persons over the age of 40 and may be asymptotic for years before progressing to rapid loss of vision. In cases where surgery is not indicated, as discussed above, topical agents, specifically beta-adrenoreceptor antagonists, have traditionally been the method of choice for treating glaucoma.

Some prostaglandins are highly effective ocular hypotensive agents and are ideally suited for the long-term medical management of glaucoma. Although the precise mechanism is not yet known, recent experimental results indicate that the prostaglandin-induced reduction in intraocular pressure results from increased uveoscleral outflow. Exemplary prostaglandins include PGF₂ₐ, PGF₁ₐ, PGE₂, and certain lipid-soluble esters, such as C₁ to C₅ alkyl esters, e.g. 1-isopropyl esters of such compounds. Certain prostaglandins, in particular PGE₂ and PGF₂ₐ, and C₁ to C₅ alkyl esters of the latter, possess ocular hypotensive activity and are recommended for use in glaucoma management. The isopropyl ester of PGF₂ₐ, for example, has been shown to have significantly greater hypotensive potency than the parent compound, which was attributed to its more effective penetration through the cornea.

Whereas prostaglandins appear to be devoid of significant intraocular side effects, ocular surface (conjunctiva) hyperemia and foreign-body sensation have been consistently associated with the topical ocular use of such compounds, in particular PGF₂ₐ and its prodrugs, e. g. its 1-isopropyl ester, in humans. The clinical potential of prostaglandins in the management of conditions associated with increased ocular pressure, e. g. glaucoma, is greatly limited by these side effects.

Certain prostaglandins and their analogs, prodrugs and derivatives, such as the PGF₂ₐ derivative latanoprost, sold under the trademark XALATAN^{®} (Pfizer Health AB Corporation, Stockholm, Sweden), have been established as compounds useful in treating ocular hypertension and glaucoma. However, latanoprost, the first prostaglandin approved by the United States Food and Drug Administration for this indication, is a prostaglandin derivative possessing the undesirable side effect of producing an increase in brown pigment in the iris of 5-15% of human eyes. The change in color results from an increased number of melanosomes (pigment granules) within iridial melanocytes. While it is still unclear whether this effect has additional and deleterious clinical ramifications, from a cosmetic standpoint alone, such side effects are undesirable.

Certain cyclopentane heptanoic acids, for example, 2-cycloalkyl or arylalkyl compounds can also be used as ocular hypotensives. These compounds, which can properly be characterized as hypotensive lipids, are effective in treating ocular hypertension. As one example, the prostamide analog bimatoprost has been discovered to be effective in reducing intraocular pressure possibly by increasing the aqueous humor outflow of an eye. Bimatoprost is an analog of a naturally occurring prostamide. Bimatoprost is available in a topical ophthalmic solution under the tradename LUMIGAN® (Allergan, Inc., Irvine, CA). Each milliliter of the solution contains 0.3 mg of bimatoprost as the active agent, 0.05 mg of benzalkonium chloride (BAK) as a preservative, and sodium chloride, sodium phosphate, dibasic, citric acid, and purified water as inactive agents.

Additionally, the rapid elimination of retinally cleared compounds makes formulation of controlled delivery systems challenging. For example, prostaglandins, in addition to their ocular side effects, may possess extremely short intraocular half-lives, and thus, may pose a challenge to the formulation of controlled delivery systems. Further, prostaglandins given by intraocular administration, let alone intraocular implants, are very rare and quite difficult to formulate.

It would be advantageous to provide implantable intracameral therapeutic agent delivery devices and systems to an eye, including intraocular implants, and methods of using such devices and systems that are capable of releasing a therapeutic bioactive agent at a sustained or controlled rate for extended periods of time and in amounts with few or no negative side effects.

The present description generally relates to intracameral therapeutic agent delivery devices and systems and therapeutic uses of such devices and systems. In particular, the present description relates to intracameral, sustained release therapeutic bioactive agent delivery systems for treatment of ocular diseases and conditions.

US 2007/0293872 A1 discloses an ocular fluid-draining device to remove fluid from the eye, for example, in a patient after glaucoma surgery. US 2009/0227933 A1 discloses methods and devices for delivering therapeutic substances into the eye.

### SUMMARY

The present description generally provides intracameral sustained release therapeutic agent delivery devices for the treatment of ocular diseases and conditions. The devices can release at least one therapeutic bioactive agent over a relatively long period of time, for example, for at least about one week or for example, between one week and one year, such as over two weeks, one month, two months or over three months or longer, after intracameral placement. Such extended release times facilitate successful treatment results. In addition, intracameral placement provides both a high, local therapeutic level of at least one therapeutic bioactive agent at the target tissue and importantly eliminates or substantially eliminates presence of toxic intermediates and metabolites at the site of the target tissue.

In one embodiment described herein are intracameral therapeutic agent delivery devices comprising: a substantially cylindrical structure for at least partial placement into the anterior chamber of an eye having a first end, a second end and at least one channel traversing the substantially cylindrical structure, the structure formed of at least one biocompatible non-biodegradable material and having at least one protrusion on an outside surface that prevents migration of the device once implanted and wherein at least one of the first end and the second ends is beveled; a sustained release material associated with at least a portion of the at least one channel; and at least one therapeutic bioactive agent associated with the sustained release material.

Further described herein are methods of treating an ocular condition comprising the steps of: providing an intracameral therapeutic agent delivery device as described herein; implanting the intracameral therapeutic agent delivery device in an eye wherein at least a portion of the intracameral therapeutic agent delivery device extends into the anterior chamber of the eye; allowing sufficient time for the therapeutic bioactive agent to migrate from the sustained release material out of the intracameral therapeutic agent delivery device and into the aqueous humor of the eye; and thereby treating the ocular condition.

In another example, the methods further include the use of an applicator to perform the implanting step. In other examples, sufficient time for the therapeutic bioactive agent to migrate from the sustained release material, out of the intracameral therapeutic agent delivery device and into the aqueous humor of the eye is between about 1 month and about 6 months.

The first end and second end is sealed with a cap such that both ends are capped. The cap or caps can be pierceable using a needle, (such as a needle attached to a syringe) and the cap or caps can be formed of a material such as, but not limited to, silicone. A syringe and associated needle can be used, for example, to fill the at least one channel of the intracameral therapeutic agent delivery device with sustained release material comprising at least one therapeutic bioactive agent.

In another embodiment, the at least one biocompatible non-biodegradable material forming the substantially cylindrical structure is a non-biodegradable polymer, a metal, a metal alloy or a combination thereof. The sustained release material can be a biodegradable polymer or a polymer matrix. The sustained release material can be packed into said at least one channel as an implant or coated on the inside surface of said at least one channel.

In one embodiment, the devices include at least one additional channel traversing the substantially cylindrical structure. The second channel can be, for example, an aqueous shunt.

The devices can further include at least one therapeutic bioactive agent releasing pore extending from inside the at least one channel to an environment outside the intracameral therapeutic agent delivery device. In various embodiments, the least one therapeutic bioactive agent releasing pore is disposed circumferentially, and in some embodiments symmetrically, around the substantially cylindrical structure of the device, or can be located on only a portion (e.g. starting at and ending, radially, within a continuous 180° degree portion/sweep) of the substantially cylindrical structure of the device of the instant disclosure. In one embodiment, the therapeutic bioactive agent migrates from the sustained release material, out of the intracameral therapeutic agent delivery device and into the aqueous humor of the eye through the at least one therapeutic bioactive agent releasing pore extending from inside the at least one channel to an environment outside the intracameral therapeutic agent delivery device.

Ocular conditions treated using the devices and methods described herein are, for example and not limited to, selected from the group consisting of aphakia, pseudophakia, astigmatism, blepharospasm, cataract, conjunctival diseases, conjunctivitis, corneal diseases, corneal ulcer, dry eye syndromes, eyelid diseases, lacrimal apparatus diseases, lacrimal duct obstruction, myopia, presbyopia, pupil disorders, refractive disorders, strabismus, glaucoma, ocular hypertension and combinations thereof.

### BRIEF DESCRIPTION OF THE DRAWINGS

Aspects of the present description are illustrated by the following drawings.
Figure 1 is a cross-sectional view of an eye illustrating its features.
Figures 2A and 2B illustrate an exemplary intracameral therapeutic agent delivery device.
Figures 3A and 3B illustrate an alternate exemplary intracameral therapeutic agent delivery device. In figure 3A, the device contains the ability to bypass aqueous from the anterior chamber to the subconjunctival space.
Figure 4 illustrates yet another exemplary intracameral therapeutic agent delivery device.
Figure 5 illustrates an exemplary implantation location for the intracameral therapeutic agent delivery devices described herein.
Figure 6 is an enlarged view of the region outlined in Figure 5 further illustrating the exemplary implantation location.
Figure 7 illustrates an intracameral therapeutic agent delivery device as described herein including a reservoir.
Figure 8 illustrates a further exemplary intracameral therapeutic agent delivery device.
Figure 9 illustrates an exemplary implantation location for the intracameral therapeutic agent delivery device illustrated in Figure 8.
Figure 10 illustrates the use of a needle to fill an intracameral therapeutic agent delivery device.
Figures 11A-D illustrate a series of Heidelberg retina angiograph (HRA) images of diffusion from an implanted device. Figures 11 A-D illustrate HRA images after 2 minutes, 5 minutes, 10 minutes and 20 minutes.

### DEFINITION OF TERMS

Certain terms as used in the specification are intended to refer to the following definitions, as detailed below. Where the definition of terms departs from the commonly used meaning of the term, applicant intends to utilize the definitions provided below, unless specifically indicated.

As used herein, "about" means plus or minus about ten percent of a number, parameter or characteristic described herein.

As used herein "biocompatible" shall mean any material that does not cause injury or death or induce an adverse reaction when placed in intimate contact with the implanted tissues. Adverse reactions include inflammation, infection, fibrotic tissue formation, cell death, or thrombosis.

As used herein, "biodegradable polymer" means a polymer or polymers which degrade *in vivo*, and wherein erosion of the polymer or polymers over time occurs concurrent with, or subsequent to, release of a drug or therapeutic bioactive agent. The terms "biodegradable" and "bioerodible" are equivalent and are used interchangeably herein. A biodegradable polymer may be a homopolymer, a copolymer, or a polymer comprising more than two different polymeric units. The polymer can be a gel or hydrogel type polymer, or mixtures or derivatives thereof.

As used herein, "ocular region" or "ocular site" means any area of the eyeball, including the anterior and posterior segment of the eye, and which generally includes, but is not limited to, any functional (e.g., for vision) or structural tissues found in the eyeball, or tissues or cellular layers that partly or completely line the interior or exterior of the eyeball. Specific examples of areas of the eyeball in an ocular region include the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina.

As used herein, "ocular condition" means a disease, ailment or condition which affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball.

For example, an anterior ocular condition is a disease, ailment or condition which affects or which involves an anterior (i.e. front of the eye) ocular region or site, such as a periocular muscle, an eye lid or an eye ball tissue or fluid which is located anterior to the posterior wall of the lens capsule or ciliary muscles. Thus, an anterior ocular condition primarily affects or involves the conjunctiva, the cornea, the anterior chamber, the iris, the posterior chamber (behind the retina but in front of the posterior wall of the lens capsule), the lens or the lens capsule and blood vessels and nerve which vascularize or innervate an anterior ocular region or site.

Thus, an anterior ocular condition can include a disease, ailment or condition, such as for example, aphakia; pseudophakia; astigmatism; blepharospasm; cataract; conjunctival diseases; conjunctivitis; corneal diseases;, corneal ulcer; dry eye syndromes; eyelid diseases; lacrimal apparatus diseases; lacrimal duct obstruction; myopia; presbyopia; pupil disorders; refractive disorders and strabismus. Glaucoma can also be considered to be an anterior ocular condition because a clinical goal of glaucoma treatment can be to reduce a hypertension of aqueous fluid in the anterior chamber of the eye (i.e. reduce intraocular pressure).

Alternatively, a posterior ocular condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular region or site.

Thus, a posterior ocular condition can include a disease, ailment or condition, such as for example, acute macular neuroretinopathy; Behcet's disease; choroidal neovascularization; diabetic uveitis; histoplasmosis; infections, such as fungal or viral-caused infections; macular degeneration, such as acute macular degeneration, non-exudative age related macular degeneration and exudative age related macular degeneration; edema, such as macular edema, cystoid macular edema and diabetic macular edema; multifocal choroiditis; ocular trauma which affects a posterior ocular site or location; ocular tumors; retinal disorders, such as central retinal vein occlusion, diabetic retinopathy (including proliferative diabetic retinopathy), proliferative vitreoretinopathy (PVR), retinal arterial occlusive disease, retinal detachment, uveitic retinal disease; sympathetic opthalmia; Vogt Koyanagi-Harada (VKH) syndrome; uveal diffusion; a posterior ocular condition caused by or influenced by an ocular laser treatment; posterior ocular conditions caused by or influenced by a photodynamic therapy, photocoagulation, radiation retinopathy, epiretinal membrane disorders, branch retinal vein occlusion, anterior ischemic optic neuropathy, non-retinopathy diabetic retinal dysfunction, retinitis pigmentosa, and glaucoma. Glaucoma can be considered a posterior ocular condition because the therapeutic goal is to prevent the loss of or reduce the occurrence of loss of vision due to damage to or loss of retinal cells or optic nerve cells (i.e. neuroprotection).

As used herein "prodrug" refers to a compound which is converted to a therapeutically active compound after administration, and the term should be interpreted as broadly herein as is generally understood in the art. While not intending to limit the scope of the present description, conversion may occur, for example, by hydrolysis of an ester group or some other biologically labile group. Generally, but not necessarily, a prodrug is inactive or less active than the therapeutically active compound to which it is converted. Ester prodrugs of the compounds disclosed herein are contemplated. An ester may be derived from a carboxylic acid of C₁ (i.e. the terminal carboxylic acid of a natural prostaglandin), or an ester may be derived from a carboxylic acid functional group on another part of the molecule, such as on a phenyl ring. While not intending to be limiting and as an example, an ester may be an alkyl ester, an aryl ester, or a heteroaryl ester.

As used herein "sustained release" refers to the release of at least one therapeutic bioactive agent, or drug from sustained release material, e.g. biodegradable polymer, at a predetermined rate. Sustained release, or controlled release, implies that the therapeutic bioactive agent is not released from the sustained release material sporadically in an unpredictable fashion and does not "burst" from the sustained release material upon contact with a biological environment (also referred to herein as first order kinetics) unless specifically intended to do so. However, the term "sustained release" as used herein does not preclude a "burst phenomenon" associated with deployment. In some example embodiments according to the present description an initial burst of at least one therapeutic bioactive agent may be desirable followed by a more gradual release thereafter. The release rate may be steady state (commonly referred to as "timed release" or zero order kinetics), that is the at least one therapeutic bioactive agent is released in even amounts over a predetermined time (with or without an initial burst phase) or may be a gradient release. A gradient release implies that the concentration of therapeutic bioactive agent released from the sustained release material changes over time. Sustained release my provide delivery of a therapeutic bioactive agents for up to about 1 month, up to about 3 months, up to about 6 months, up to about 1 year or up to about 5 years or longer.

As used herein, "therapeutically effective amount" means a level or amount of a therapeutic bioactive agent or drug needed to treat an ocular condition, or reduce or prevent ocular injury or damage without causing significant negative or adverse side effects to the eye or a region of the eye. In view of the above, a therapeutically effective amount of a bioactive agent is an amount that is effective in reducing at least one symptom of an ocular condition.

### DETAILED DESCRIPTION OF THE INVENTION

Described herein are intracameral therapeutic agent delivery devices and methods for sustained release and hence delivery of at least one therapeutic bioactive agent in an eye, for example, the anterior chamber, for treatment of one or more ocular conditions or diseases. The intracameral therapeutic agent delivery devices can treat an ocular disease or condition by attaining a sustained or controlled release of at least one therapeutic bioactive agent from an implant associated with the intracameral therapeutic agent delivery devices described herein. The intracameral therapeutic agent delivery devices are effective in treating or reducing at least one symptom of an ocular disease or condition, such as by increasing macular thickness, reducing retinal edema, reducing retinal vein occlusion, treating ocular hypertension or hypotension, reducing at least one symptom of glaucoma and/or by maintaining or improving visual acuity and color vision, for example.

In one embodiment, the sustained release of therapeutic bioactive agents is accomplished using an intracameral therapeutic agent delivery device having substantially cylindrical structures for at least partial placement into the anterior chamber of an eye.

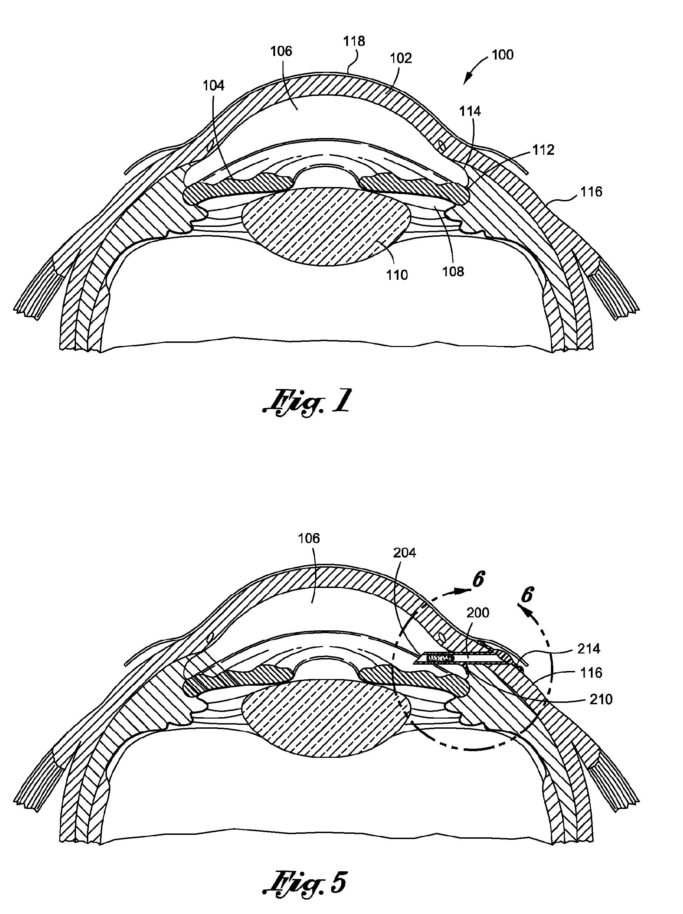

Referring to Figure 1, particular ocular features of eye **100** are indicated. Eye **100** includes cornea **102** and iris **104**, which surround anterior chamber **106**. Behind iris **104** is the posterior chamber **108** including natural crystalline lens **110**. Anterior chamber **106** includes iridocorneal angle **112** and trabecular meshwork **114**. Covering cornea **102** and sclera **116** partially is conjunctival sac **118**.

In one embodiment, the intracameral therapeutic agent delivery devices have a first end, a second end and at least one channel traversing the substantially cylindrical structure that makes up the body of the devices. Further and in one embodiment, the substantially cylindrical structures are formed of at least one biocompatible non-biodegradable material and have at least one protrusion on an outside surface that prevents migration of said device once implanted and one of the ends of the substantially cylindrical structure is beveled. The at least one protrusion can be provided alone or in conjunction with ribs 212, of which ribs 212 and the at least one protrusion 210 can extend the same distance from the outer surface of the intracameral therapeutic agent delivery device, or where one or the other of ribs 212 or the at least one protrusion 210 can extend a further distance than the other from the outer surface of the intracameral therapeutic agent delivery device.

A sustained release material is associated with at least a portion of a channel of the substantially cylindrical structure. Further, at least one therapeutic bioactive agent is associated with the sustained release material.

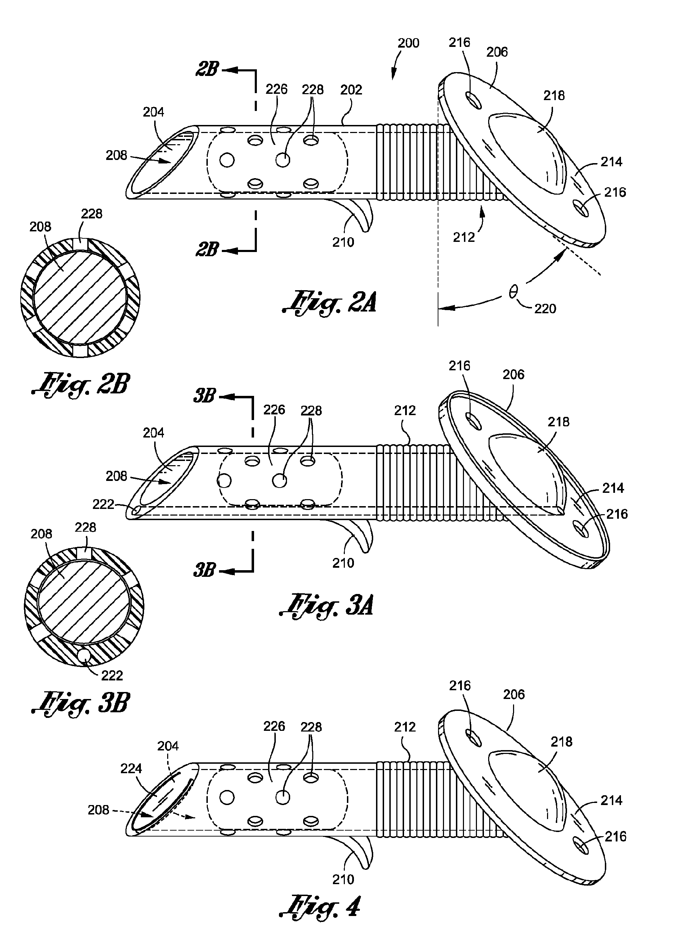

Figures 2-4 illustrate exemplary intracameral therapeutic agent delivery devices according to the present description. In one example, an intracameral therapeutic agent delivery device **200** includes substantially cylindrical structure **202** having first end **204**, second end **206** and tube **208** traversing the device. The device can also include at least one protrusion **210** for anchoring the device in place once implanted into a tissue. Also, in a particular configuration, first end **204** is beveled allowing insertion of first end **204** into one or more tissues. To further aid in anchoring the device within a tissue, ribs **212** are included near second end **206** to interact with the surrounding tissues.

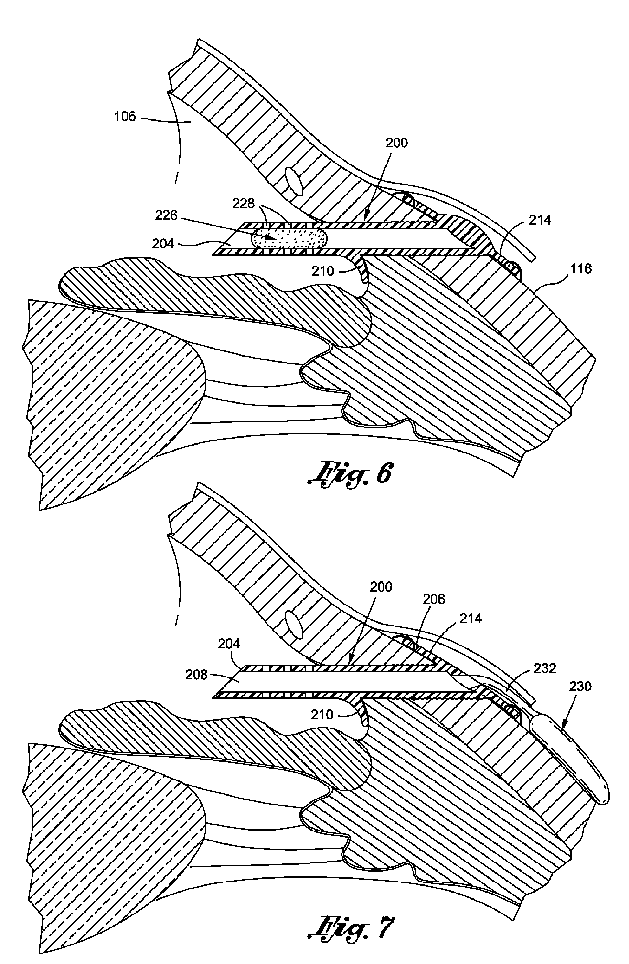

Second end **206** can have head portion **214** having one or more suture holes **216** wherein sutures or other anchoring devices can be threaded. Second end **206** can also include optional plug **218** to plug tube **208**. Head portion **214** can be provided at any angle θ **220** that will accommodate implantation on, at or through a given tissue. In one example embodiment, intracameral therapeutic agent delivery device **200** is implanted through cornea **102** or sclera **116** using a bevel at first end **204** so that first end **204** is within, for example, anterior chamber **106**. In such an embodiment, illustrated in Figures 5 and 6, intracameral therapeutic agent delivery device **200** is inserted through sclera **116** into anterior chamber **106**. Head portion **214** abuts sclera **116** and at least one protrusion **210** anchors the device inside the anterior chamber at trabecular meshwork **114**.

As illustrated in Figure 3A and 3B, intracameral therapeutic agent delivery device **200** can further include a second tube **222** traversing the device. Second tube **222** can be sealed at one or both ends; however, in an exemplary embodiment, fluid is free to flow through second tube **222** without a barrier or plug. In one example embodiment, intracameral therapeutic agent delivery device **200** can be used to deliver a therapeutic bioactive agent as well as actively raise or lower intraocular pressure by liquid exchange through second tube **222**. The aqueous humor in the anterior chamber can pass into the subconjunctival space. To reduce post-operative fibrosis leading to filtering conjunctival bleb failure, a sustained-release coating or implant at the 214 position on figure 3a of an antifibrotic agent can be used. The anti-fibrotic agents can be but are not limited to 5-flurouracil, mitomycin-C, and placlitaxel. The sustained-release coating or implant at the 214 position would be polymer-based, for example, biodegradable polymers such as but not limited to superhydrolyzed polyvinyl alcohol, and the synthetic aliphatic polyesters of the poly-a-hydroxy acid family, which include polyglycolic acid (PGA), polylactic acid (PLA), and the PGA/PLA copolymer, polylactic-co-glycolic acid (PLGA). In addition, the sustained-release coating could use non-biodegradable polymers, such as but not limited to silicones and ethylene vinyl acetate.

Further, illustrated in Figure 4, intracameral therapeutic agent delivery device **200** can optionally have a barrier **224** at first end **204**. Optional plug **218** and barrier **224** can be made of the same material or a different material. Those materials can include, but are not limited to, pierceable polymers such as silicone, biodegradable polymers, biodegradable metals, non-biodegradable polymers or metals such as those used to form the device itself, and even a hinged member, such as a door. In particular embodiments, an in-line valve within the substantially cylindrical structure **202** can be provided and utilized to prevent retrograde fluid flow in the intracameral therapeutic agent delivery device.

In one example embodiment, intracameral therapeutic agent delivery device 200 can have one or more therapeutic bioactive agents associated with tube **208** in at least region **226**. Region **226** can have at least one pore **228** where through therapeutic bioactive agents can pass into the surrounding tissues. In particular embodiments, region 226, that is where through therapeutic bioactive agents can pass into surrounding tissues, can be from about 25% to about 75%, or from about 25% to about 60%, or from about 35% to about 50% of the length of substantially cylindrical structure **202** having first end **204**, second end **206** and tube **208** traversing the device. At least one pore **228** can have diameters ranging from about 1µm to about 0.1 mm.

Further, the therapeutic bioactive agents can be supplied in a sustained release formulation and coated onto at least a portion of the surface of intracameral therapeutic agent delivery device **200**. In one example embodiment, intracameral therapeutic agent delivery device **200** is coated inside tube **208**. Coating the inside tube **208** can be from about 10% to about 90%, from about 20% to about 80%, from about 30% to about 70% or from about 40% to about 60% of the internal surface inside tube **208**.

In one embodiment and as seen in Figure 7, intracameral therapeutic agent delivery device **200** can further include reservoir **230** wherein one or more therapeutic bioactive agent is housed for delivery through tube **208** traversing the device. Transfer tube **232** can extend from reservoir **230** to second end **206** and take the place of optional plug **218**. Reservoir **230** can take any form known in the art and can be actuated by a means such as a microchip (not shown) or pressure applied thereto. In such an embodiment, therapeutic bioactive agents can be extruded from reservoir **230**, through transfer tube **232**, through tube **208** and into anterior chamber **106** through first end **204**. In an alternate environment, first end **204** can be plugged and the therapeutic bioactive agents can exit the device through at least one pore **228**.

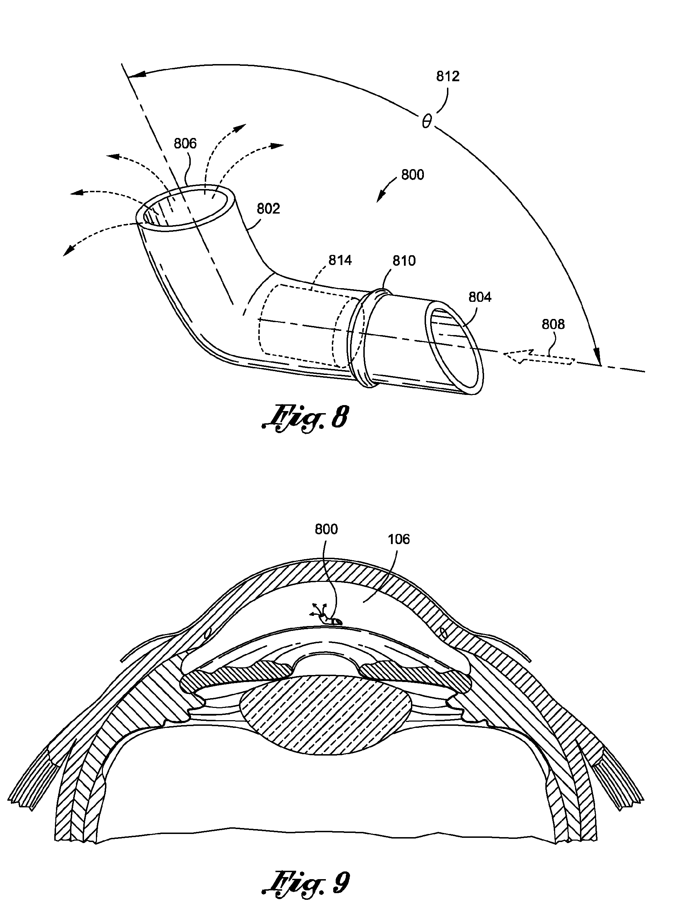

In another embodiment, illustrated in Figure 8, is intracameral therapeutic agent delivery device **800** having substantially cylindrical structure **802** having first end **804** and second end **806** and tube **808** traversing the device. The device can also include at least one protrusion **810** for anchoring the device in place once implanted into a tissue. Also, first end **804** is beveled allowing insertion of first end **804** into one or more tissues.

Substantially cylindrical structure **802** can be provided at any angle θ **812** that will accommodate implantation on, at or through a given tissue. In one example embodiment, intracameral therapeutic agent delivery device **800** is implanted into trabecular meshwork **114** or through trabecular meshwork **114** into Schlemn's canal. In such an embodiment, illustrated in Figure 9, intracameral therapeutic agent delivery device **800** is inserted into trabecular meshwork **114** and provides sustained release of at least one therapeutic bioactive agent from the device into anterior chamber **106**.

In another embodiment, intracameral therapeutic agent delivery device **800** can have one or more therapeutic bioactive agents associated with tube **808** in at least region **814**. Region **814** can contain therapeutic bioactive agents that pass into the surrounding tissues through second end **806**. In other embodiments, therapeutic bioactive agents can pass through one or more pores (not shown) if second end **806** is sealed. In some embodiments, first end **804** can be sealed or unsealed depending on the device itself and location of implantation.

In one aspect, an intracameral therapeutic agent delivery device provided in accordance with the present disclosure can have a wall thickness of about 0.051 mm (0.002 inch), which allows for maximal inner lumen area for sustained release materials, including in the form of an implant, for loading into or coated onto at least a portion, such as the inner surface, of an intracameral therapeutic agent delivery device provided in accordance with the present disclosure.

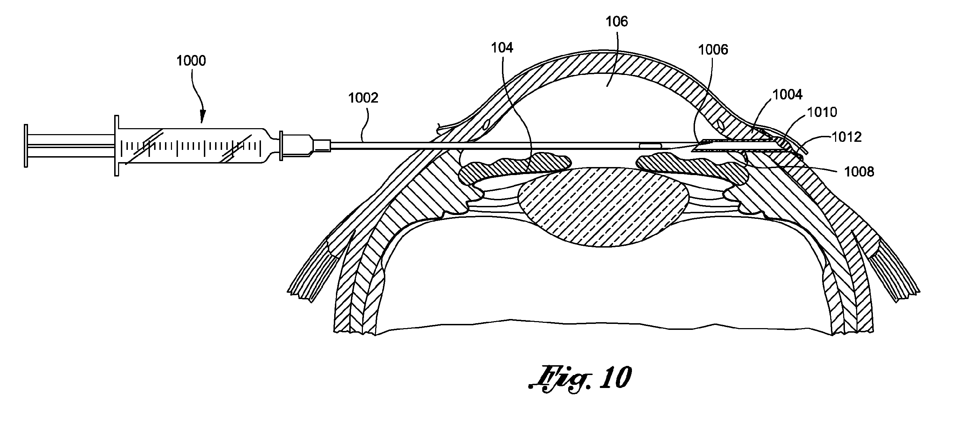

In one embodiment, sustained release materials in the form of implants, for example, can be loaded into or coated on at least a portion of an intracameral therapeutic agent delivery device as described herein as an initial loading of therapeutic bioactive agent or as a "refill" when previous amounts of therapeutic bioactive agent have been substantially depleted. As illustrated in Figure 10 and as one exmaple, syringe **1000** is fitted with needle **1002**. Intracameral therapeutic agent delivery device **1004** includes pierceable silicone end cap **1006**. Needle **1002** is passed through anterior chamber **106**, parallel to iris **104**, through pierceable silicone end cap **1006** and into the substantially cylindrical structure **1008** of intracameral therapeutic agent delivery device **1004**. Once needle **1002** has breached pierceable silicone end cap **1006**, sustained release materials can be injected into intracameral therapeutic agent delivery device **1004**. Upon completion of injection, needle **1002** is removed, pierceable silicone end cap **1006** reseals itself and at least one therapeutic bioactive agent begins to provide sustained therapy to the eye.

Alternatively, needle **1004** in Figure 10 can be inserted through second pierceable silicone end cap **1010** located at second end **1012**. In this embodiment, minimal ocular tissue is intercepted and patient discomfort is minimal. The method used to deliver sustained release materials to intracameral therapeutic agent delivery devices described herein are at the discretion of the attending physician.

Important to the sustained release provided by the implants and sustained release coatings associated with the intracameral therapeutic agent delivery devices, is the relative average molecular weight of the polymers chosen to form the implants and coatings. Molecular weight of a polymer is mathematically related to the polymers mean viscosity, and therefore, different molecular weights of the two partitioned phases or polymers are included to modulate the release profile.

Suitable polymers for use in forming the implants and/or sustained release formulations associated with the intracameral therapeutic agent delivery devices described herein include those which are biocompatible with the eye so as to cause no substantial interference with the functioning or physiology of the eye. Such polymers preferably are at least partially, and more preferably, substantially completely biodegradable or bioerodible.

The polymers may be addition or condensation polymers. Generally, besides carbon and hydrogen, the polymers can include at least one oxygen. The oxygen may be present as oxy, e.g. hydroxy or ether, carbonyl, e.g. non-oxo-carbonyl, such as carboxylic acid ester, and the like.

Useful bioerodible polymers include poly(D,L-lactide-co-glycolide) (PLGA), poly(D,L-lactide) (PLA), polyesters, poly(ortho ester), poly(phosphazine), poly (phosphate ester), poly(ε-caprolactone) (PCL), natural polymers such as gelatin or collagen, or polymeric blends.

In certain embodiments, PLGA is used and the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the resulting polymer. The percentage of polylactic acid in the PLGA copolymer can be 0-100%, preferably about 15-85%, more preferably about 35-65%. In some exemplary implants, a 50:50 PLGA copolymer is used.

Some preferred characteristics of the polymers or polymeric materials for use in the implants and/or sustained release formulations described herein may include biocompatibility with the selected therapeutic bioactive agent, ease of use of the polymer in making the therapeutic agent delivery systems, a half-life in the physiological environment of at least about 6 hours, preferably greater than about one day, and water insolubility.

Release of at least one therapeutic bioactive agent from a biodegradable polymer, such as those described herein, is the consequence of several mechanisms or combinations of mechanisms. Some of these mechanisms include desorption from the implant surface, dissolution, diffusion through porous channels of the hydrated polymer and erosion. Erosion can be bulk or surface or a combination of both. In some embodiments, therapeutic bioactive agents are released for no more than about 3-30 days after administration. For example, an implant may comprise at least one therapeutic bioactive agent and the implant associated with the intracameral therapeutic agent delivery device degrades at a rate effective to sustain release of a therapeutically effective amount for about one month after being placed within the eye.

In one embodiment, the implants and/or sustained release formulations can comprise a PLA polymer having a first mean viscosity, a 50:50 PLGA polymer having a second mean viscosity and at least one therapeutic bioactive agent. In an example, the PLA polymer has a mean viscosity between about 0.25 and about 0.35 dl/g or about 1.3 dl/g and about 1.7 dl/g and the PLGA polymer has a mean viscosity between about 0.16 and about 0.44 dl/g. The mean viscosities identified above may be determined in 0.1 % chloroform at 25°C.

In a further embodiment, the implants and/or sustained release formulations comprise a PLA polymer having a first molecular weight, a 50:50 PLGA polymer having a second molecular weight and at least one therapeutic bioactive agent. The first molecular weight is at least equal to or greater than the second molecular weight. For example, the first molecular weight is at least three times greater than the second molecular weight. In other examples, the first molecular weight is at least four, five or ten times greater than the second molecular weight. The difference in molecular weight between the PLA and PLGA polymers allow the resulting implant and/or sustained release formulation to remain stable once formed and provides the *in vivo* characteristics sought. In one embodiment, the PLA polymer can have a molecular weight between about 300,000 and about 100,000 Da and the PLGA polymer can have a molecular weight between about 80,000 and about 10,000 Da.

In one embodiment, the PLA and PLGA polymers form two different phases within the implant and/or sustained release formulation and the at least one therapeutic bioactive agent partitions itself into one phase or the other. In another embodiment, the at least one at least one therapeutic bioactive agent, such as a prostaglandin, for example, partitions itself into the PLGA phase.

The implants and/or sustained release formulations associated with the intracameral therapeutic agent delivery devices described herein are developed based upon the discoveries that: (1) even though therapeutic bioactive agents can be eliminated from the eye extremely rapidly with short half-lives, it is theoretically feasible to deliver therapeutic bioactive agents to ocular tissues at therapeutic levels over a period of, for example, one week, or for a period of time between about 2 months and about a year; (2) systemic therapeutic bioactive agents can cause negative vision effects; (3) the negative vision effects of systemic administration are probably a result of metabolites generated by hepatic metabolism; (4) a method for the intraocular delivery of therapeutic bioactive agents for the treatment of intraocular diseases is feasible; (5) a method to reduce the intraocular toxicity of locally delivered therapeutic bioactive agents is feasible; and (6) compositions of bioerodible polymeric implants and/or sustained release formulations associated with intracameral therapeutic agent delivery devices as described herein, and therapeutic bioactive agents for the treatment of ocular diseases and conditions, can be prepared.

Delivery of drugs or bioactive agents to the optic nerve, retina, vitreous, subTenon's space, and uveal tract is typically achieved by high systemic dosing which can cause toxicity or toxic metabolites, intra-ocular injections or other heroic measures. Penetration of systemically administered drugs into the retina is severely restricted by the blood-retinal barriers (BRB) for most compounds. As determined herein for example, local delivery of latanoprost utilizing an intracameral therapeutic agent delivery device in accordance with the teachings of the instant disclosure can prevent systemic toxicities and mitigate the BRB problems.

The intracameral therapeutic agent delivery devices and associated implants disclosed herein can also be configured to prevent or treat diseases or conditions, such as the following: maculopathies/retinal degeneration: macular degeneration, including age related macular degeneration (ARMD), such as non-exudative age related macular degeneration and exudative age related macular degeneration, choroidal neovascularization, retinopathy, including diabetic retinopathy, acute and chronic macular neuroretinopathy, central serous chorioretinopathy, and macular edema, including cystoid macular edema, and diabetic macular edema. Uveitis/retinitis/choroiditis: acute multifocal placoid pigment epitheliopathy, Behcet's disease, birdshot retinochoroidopathy, infectious (syphilis, lyme, tuberculosis, toxoplasmosis), uveitis, including intermediate uveitis (pars planitis) and anterior uveitis, multifocal choroiditis, multiple evanescent white dot syndrome (MEWDS), ocular sarcoidosis, posterior scleritis, serpignous choroiditis, subretinal fibrosis, uveitis syndrome, and Vogt-Koyanagi-Harada syndrome. Vascular diseases/exudative diseases: retinal arterial occlusive disease, central retinal vein occlusion, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angitis, sickle cell retinopathy and other hemoglobinopathies, angioid streaks, familial exudative vitreoretinopathy, Eales disease. Traumatic/surgical: sympathetic ophthalmia, uveitic retinal disease, retinal detachment, trauma, laser, PDT, photocoagulation, hypoperfusion during surgery, radiation retinopathy, bone marrow transplant retinopathy. Proliferative disorders: proliferative vitreal retinopathy and epiretinal membranes, proliferative diabetic retinopathy. Infectious disorders: ocular histoplasmosis, ocular toxocariasis, presumed ocular histoplasmosis syndrome (POHS), endophthalmitis, toxoplasmosis, retinal diseases associated with HIV infection, choroidal disease associated with HIV infection, uveitic disease associated with HIV Infection, viral retinitis, acute retinal necrosis, progressive outer retinal necrosis, fungal retinal diseases, ocular syphilis, ocular tuberculosis, diffuse unilateral subacute neuroretinitis, and myiasis. Genetic disorders: retinitis pigmentosa, systemic disorders with associated retinal dystrophies, congenital stationary night blindness, cone dystrophies, Stargardt's disease and fundus flavimaculatus, Bests disease, pattern dystrophy of the retinal pigmented epithelium, X-linked retinoschisis, Sorsby's fundus dystrophy, benign concentric maculopathy, Bietti's crystalline dystrophy, pseudoxanthoma elasticum. Retinal tears/holes: retinal detachment, macular hole, giant retinal tear. Tumors: retinal disease associated with tumors, congenital hypertrophy of the RPE, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, intraocular lymphoid tumors. Miscellaneous: punctate inner choroidopathy, acute posterior multifocal placoid pigment epitheliopathy, myopic retinal degeneration, acute retinal pigment epithelitis and the like.

The release of therapeutic bioactive agent from an implant, out of the intracameral therapeutic agent delivery device and into the anterior chamber, may include an initial burst of release followed by a gradual increase in the amount released, or the release may include an initial delay in release, followed by an increase in release. When the implants are substantially completely degraded, the percentage of therapeutic bioactive agent that has been released is about one hundred percent. In one embodiment, the intracameral therapeutic agent delivery devices and associated implants described herein do not release substantially all, or about 100%, of the therapeutic bioactive agents, until after being placed in an eye for about one week or more.

It may be desirable to provide a relatively constant rate of release of a therapeutic bioactive agent from the intracameral therapeutic agent delivery device over the life of an implant and/or sustained release formulation. For example, it may be desirable for a therapeutic bioactive agent to be released in an amount from about 0.01 µg to about 2 µg per day for the life of the implant. However, the release rate may change to either increase or decrease depending on the formulation of the biodegradable polymer matrix. In addition, the release profile of a therapeutic bioactive agent may include one or more linear portions and/or one or more non-linear portions. Preferably, the release rate is greater than zero once the implant and/or sustained release formulation has begun to degrade or erode.

In one embodiment, therapeutic bioactive agents form about 1% to about 90% by weight of the implants and/or sustained release formulations; more preferably, from about 5% to about 30% by weight of the implants and/or sustained release formulations. In another embodiment, a therapeutic bioactive agent comprises about 10% by weight of an implant and/or sustained release formulation. In still yet another embodiment, a therapeutic bioactive agent comprises about 20% by weight of an implant and/or sustained release formulation.

In some embodiments, an implant and/or sustained release formulation associated with an intracameral therapeutic agent delivery device as described herein can release about 1% a therapeutic bioactive agent, that is, of the total therapeutic bioactive agent contained therein, per day. In a further embodiment, the implants and/or sustained release formulations may have a release rate of about 0.7% per day of the total therapeutic bioactive agent contained therein, when measured *in vitro.* Thus, in one example, over a period of about 40 days, about 30% of a therapeutic bioactive agent comprising an implant and/or sustained release formulation associated with an intracameral therapeutic agent delivery device may have been released.

The total weight of implant and/or sustained release formulation in a single dosage associated with a intracameral therapeutic agent delivery device as described herein is an amount dependent on the volume of the substantially cylindrical structure and the activity or solubility of the at least one therapeutic bioactive agent. In one embodiment, the dose is about 0.1 mg to about 200 mg per implant. For example, a single intracameral therapeutic agent delivery device may contain about 1 mg, 3 mg, or about 5 mg, or about 8 mg, or about 10 mg, or about 100 mg or about 150 mg, or about 175 mg, or about 200 mg, or any useful range/amount therebetween, of implant and/or sustained release formulation, including the incorporated therapeutic bioactive agents.

In particular embodiments, the implants disclosed herein may have a diameter size of between about 5 µm and about 1 mm, or between about 10 µm and about 0.8 mm for administration with a needle. For needle-injected implants, the implants may have any appropriate dimensions so long as the longest dimension permits the implant to move through a needle.

The implants may be of any particulate geometry including micro- and nanospheres, micro- and nano-particles, spheres, powders, rods, fragments, cubes, pills, disks, films, and the like. The upper limit for size will be determined by factors such as toleration for the implant, size limitations on insertion, desired rate of release, ease of handling, and for fitting into the intracameral therapeutic agent delivery device and taught herein. Spheres may be in the range of about 0.5 µm to 4 mm in diameter, with comparable volumes for other shaped particles.

Further, the implants may have a maximum cross-section less than about 200 µm. In certain embodiments, the implants have an average or mean cross-section less than about 50 µm. In further embodiments, the cross-section ranges from about 30 µm to about 50 µm. The diameters of the implants can range from 0.1 µm to 1 mm.

The implants, in one embodiment, can be formed as films to be inserted into the intracameral therapeutic agent delivery devices described herein. In order to reduce the overall dimensions of the implants when formed as films, they can be rolled up and inserted into the substantially cylindrical structure of the intracameral therapeutic agent delivery devices. Once inserted, the rolled up films can unroll either fully or partially within the confines of the devices, causing no damage to the intracameral therapeutic agent delivery devices themselves or to surrounding ocular tissue.

The size and form of the implant can also be used to control the rate of release, period of treatment, and therapeutic bioactive agent concentration at the site of intracameral therapeutic agent delivery device placement. Larger implants will deliver a proportionately larger dose, but depending on the surface to mass ratio, may have a slower release rate. The particular size and geometry of the implant is chosen to suit the activity of the therapeutic agent and the location of its target tissue.

The proportions of therapeutic bioactive agent, polymer, and any other modifiers may be empirically determined by formulating several implant batches with varying average proportions. A United States Pharmacopeia (USP) approved method for dissolution or release test can be used to measure the rate of release (USP 23; NF 18 (1995) pp. 1790-1798). For example, using the infinite sink method, a weighed sample of implants is added to a measured volume of a solution containing 0.9% NaCl in water, where the solution volume will be such that the therapeutic bioactive agent concentration after release is less than 5% of saturation. The mixture is maintained at 37°C and stirred slowly to maintain the implants in suspension. The appearance of the dissolved therapeutic bioactive agent as a function of time may be followed by various methods known in the art, such as spectrophotometrically, HPLC, mass spectroscopy, etc. until the absorbance becomes constant or until greater than 90% of the therapeutic bioactive agent has been released.

The at least one therapeutic bioactive agent can be any ophthalmically acceptable therapeutic agent or drug. For example, therapeutic bioactive agents include antihistamines, antibiotics, beta blockers, steroids, antineoplastic agents, immunosuppressive agents, antiviral agents, antioxidant agents, and mixtures thereof. In other embodiments, multiple implants can be used with two or more different therapeutic bioactive agents.

Prostaglandins can also be used as therapeutic bioactive agents. "Prostaglandin" as used herein is meant to include one or more types of prostaglandin derivatives, prostaglandin analogues including prostamides and prostamide derivatives, prodrugs, salts thereof, and mixtures thereof. In certain embodiments, the prostaglandin comprises a compound having the structure 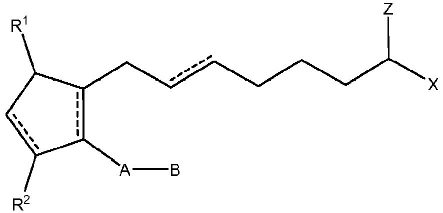 wherein the dashed bonds represent a single or double bond which can be in the *cis* or *trans* configuration; A is an alkylene or alkenylene radical having from two to six carbon atoms, which radical may be interrupted by one or more oxide radicals and substituted with one or more hydroxy, oxo, alkyloxy or akylcarboxy groups wherein the alkyl radical comprises from one to six carbon atoms; B is a cycloalkyl radical having from three to seven carbon atoms, or an aryl radical, selected from hydrocarbyl aryl and heteroaryl radicals having from four to ten carbon atoms wherein the heteroatom is selected from nitrogen, oxygen and sulfur atoms; X is-OR⁴ or -N(R⁴)₂ wherein R⁴ is selected from hydrogen, a lower alkyl radical having from one to six carbon atoms, 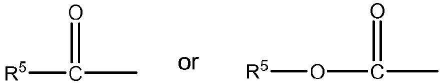 wherein R⁵ is a lower alkyl radical having from one to six carbon atoms; Z is =0 or represents two hydrogen radicals; one of R¹ and R² is =0, -OH or a -O(CO)R⁶ group, and the other one is -OH or -O(CO)R⁶, or R¹ is =0 and R² is hydrogen, wherein R⁶ is a saturated or unsaturated acyclic hydrocarbon group having from 1 to about 20 carbon atoms, or -(CH2)ₘR⁷ wherein m is 0 or an integer of from 1 to 10, and R⁷ is cycloalkyl radical, having from three to seven carbon atoms, or a hydrocarbyl aryl or heteroaryl radical, as defined above, or a pharmaceutically acceptable salt thereof.

Pharmaceutically acceptable acid addition salts of the compounds described are those formed from acids which form non-toxic addition salts containing pharmaceutically acceptable anions, such as the hydrochloride, hydrobromide, hydroiodide, sulfate, or bisulfate, phosphate or acid phosphate, acetate, maleate, fumarate, oxalate, lactate, tartrate, citrate, gluconate, saccharate and p-toluene sulphonate salts.

In one embodiment, a prostaglandin having the structure 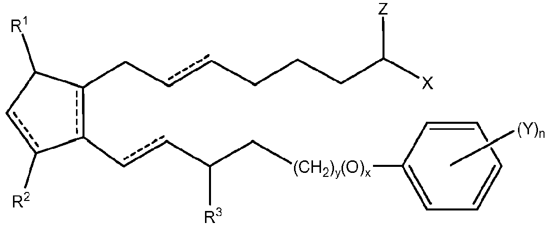 wherein y is 0 or 1, x is 0 or 1 and x and y are not both 1, Y is selected the group consisting of alkyl, halo, nitro, amino, thiol, hydroxy, alkyloxy, alkylcarboxy and halo substituted alkyl, wherein said alkyl radical comprises from one to six carbon atoms, n is 0 or an integer of from 1 to 3 and R³ is =0, -OH or O(CO)R⁶ can be used.

In additional embodiments, the prostaglandin has the formula 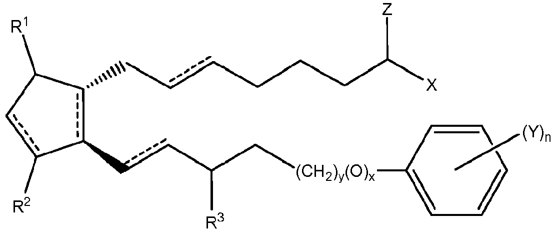 wherein hatched lines indicate the *alpha* configuration and solid triangles indicate the *beta* configuration.

The prostaglandin can also have the formula 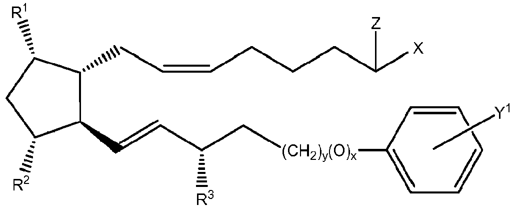 wherein Y¹ is Cl or trifluoromethyl.

Other prostaglandins can have the following formula 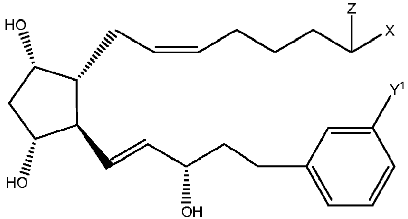 and 9-, 11- and/or 15 esters thereof.

In one embodiment, the prostaglandin component comprises a compound having the formula

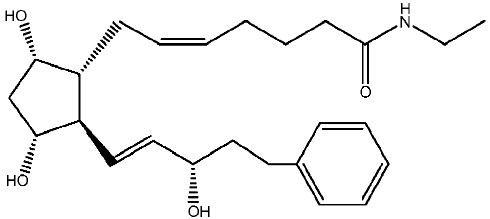

This compound is also known as bimatoprost and is publicly available in a topical ophthalmic solution under the tradename, LUMIGAN^{®} (Allergan, Inc., Irvine, CA). Prostamide analogues can be used, such but not limited to bimatoprost acid, bimatoprost salt, and 17 dichlorophenyl prostamide.

In another embodiment of an intraocular implant, the prostaglandin comprises a compound having the structure

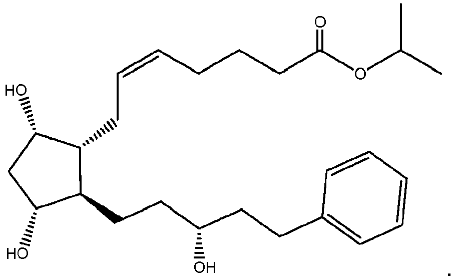

This prostaglandin is known as latanoprost and is publicly available in a topical ophthalmic solution under the tradename, XALATAN^{®}. Thus, the implants and/or sustained release formulations may comprise at least one therapeutic bioactive agent which comprises, consists essentially of, or consists of latanoprost, a salt thereof, isomer, prodrug or mixtures thereof.

Additional therapeutic bioactive agents or drugs include, without limitation, those disclosed in U.S. Pat. No. 4,327,725, columns 7-8, and the entire disclosure of which is incorporated herein by reference for all that it discloses regarding pharmacologic or therapeutic agents. Additional examples of useful therapeutic bioactive agents or drugs are discussed below.

Examples of useful antihistamines for use in accordance with the instant disclosure include, but are not limited to, loradatine, hydroxyzine, diphenhydramine, chlorpheniramine, brompheniramine, cyproheptadine, terfenadine, clemastine, triprolidine, carbinoxamine, diphenylpyraline, phenindamine, azatadine, tripelennamine, dexchlorpheniramine, dexbrompheniramine, methdilazine, and trimprazine doxylamine, pheniramine, pyrilamine, chiorcyclizine, thonzylamine, and derivatives thereof.

Examples of useful antibiotics include without limitation, cefazolin, cephradine, cefaclor, cephapirin, ceftizoxime, cefoperazone, cefotetan, cefutoxime, cefotaxime, cefadroxil, ceftazidime, cephalexin, cephalothin" cefamandole, cefoxitin, cefonicid, ceforanide, ceftriaxone, cefadroxil, cephradine, cefuroxime, ampicillin, amoxicillin, cyclacillin, ampicillin, penicillin G, penicillin V potassium, piperacillin, oxacillin, bacampicillin, cloxacillin, ticarcillin, azlocillin, carbenicillin, methicillin, nafcillin, erythromycin, tetracycline, doxycycline, minocycline, aztreonam, chloramphenicol, ciprofloxacin hydrochloride, clindamycin, metronidazole, gentamicin, lincomycin, tobramycin, vancomycin, polymyxin B sulfate, colistimethate, colistin, azithromycin, augmentin, sulfamethoxazole, trimethoprim, and derivatives thereof.

Examples of beta blockers for use in accordance with the instant disclosure include, but are not limited to, acebutolol, atenolol, labetalol, metoprolol, propranolol, timolol, and derivatives thereof. Examples of steroids include corticosteroids, such as cortisone, prednisolone, flurometholone, dexamethasone, medrysone, loteprednol, fluazacort, hydrocortisone, prednisone, betamethasone, prednisone, methylprednisolone, riamcinolone hexacatonide, paramethasone acetate, diflorasone, fluocinonide, fluocinolone, triamcinolone, derivatives thereof, and mixtures thereof.

Examples of antineoplastic drugs include, but are not limited to, adriamycin, cyclophosphamide, actinomycin, bleomycin, duanorubicin, doxorubicin, epirubicin, mitomycin, methotrexate, fluorouracil, carboplatin, carmustine (BCNU), methyl-CCNU, cisplatin, etoposide, interferons, camptothecin and derivatives thereof, phenesterine, taxol and derivatives thereof, taxotere and derivatives thereof, vinblastine, vincristine, tamoxifen, etoposide, piposulfan, cyclophosphamide, and flutamide, and derivatives thereof.

Examples of immunosuppressive drugs include, but are not limited to, cyclosporine, azathioprine, tacrolimus, and derivatives thereof.

Examples of antiviral agents include but are not limited to, interferon gamma, zidovudine, amantadine hydrochloride, ribavirin, acyclovir, valciclovir, dideoxycytidine, phosphonoformic acid, ganciclovir, and derivatives thereof.

Examples of antioxidants include but are not limited to, ascorbate, alpha-tocopherol, mannitol, reduced glutathione, various carotenoids, cysteine, uric acid, taurine, tyrosine, superoxide dismutase, lutein, zeaxanthin, cryotpxanthin, astazanthin, lycopene, N-acetyl-cysteine, carnosine, gamma-glutamylcysteine, quercitin, lactoferrin, dihydrolipoic acid, citrate, Ginkgo Biloba extract, tea catechins, bilberry extract, vitamins E or esters of vitamin E, retinyl palmitate, and derivatives thereof.

Other therapeutic agents include squalamine, carbonic anhydrase inhibitors, alpha-2 adrenergic receptor agonists, antiparasitics, antifungals, beta-adrenergic receptor antagonists such as timolol maleate, carbonic anyhdrase inhibitors such as dorzolamide, and derivatives thereof. Combinations of any of the drugs and bioactive agents mentioned can be used according to the present description.

The amount of therapeutic bioactive agent or additional bioactive agent or drug employed in the implants and/or sustained release formulations, will vary widely depending on the effective dosage required and the desired rate of release. The therapeutic agent is at least about 1% (w/w), or at least about 10 % (w/w) of the implant and/or sustained release formulation, and in particular embodiments, up to about 40% (w/w), or up to about about 50 % (w/w).

In addition to the therapeutic agents and drugs, the implants disclosed herein may include or may be provided in the sustained release formulations described herein, can include effective amounts of buffering agents, preservatives and the like. Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents advantageously are present in amounts sufficient to maintain a pH of the system of between about 2 to about 9 and more preferably about 4 to about 8. As such, the buffering agent may be as much as about 5% by weight of the total implant. Suitable water soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from about 0.001% to about 5% by weight and preferably about 0.01% to about 2% by weight of the implant and/or sustained release formulations. In one example embodiment, a benzylalkonium chloride preservative is provided in the sustained release formulations.

In other embodiments, mixtures of controlled release profiles within a single implant and/or sustained release formulation or within several different implants may be utilized employing the same or different therapeutic bioactive agents. In this way, a cocktail of release profiles, giving a biphasic or triphasic release with a single intracameral therapeutic agent delivery device is achieved, where the pattern of release may be greatly varied.

Additionally, release modulators such as those described in U. S. Patent No. 5,869,079 may be included in the sustained release formulations. The amount of release modulator employed will be dependent on the desired release profile, the activity of the modulator, and on the release profile of therapeutic bioactive agent in the absence of modulator. Electrolytes such as sodium chloride and potassium chloride may also be included in the formulations. Where the buffering agent or enhancer is hydrophilic, it may also act as a release accelerator. Hydrophilic additives act to increase the release rates through faster dissolution of the material surrounding the bioactive agent, which increases the surface area of the bioactive agent exposed, thereby increasing the rate of bioactive agent bioerosion. Similarly, a hydrophobic buffering agent or enhancer dissolves more slowly, slowing the exposure of bioactive agent, and thereby slowing the rate of bioactive agent bioerosion.

Various techniques may be used in producing the implants described herein. Useful techniques include, but are not necessarily limited to, self-emulsification methods, super critical fluid methods, solvent evaporation methods, phase separation methods, spray drying methods, grinding methods, interfacial methods, molding methods, injection molding methods, extrusion methods, combinations thereof and the like.

Generally, the processes for making the implants involve dissolving the appropriate polymers and bioactive agents in a solvent. Solvent selection will depend on the polymers and bioactive agents chosen. For the implants described herein, including a bioactive agent such as latanoprost, dichloromethane (DCM) is an appropriate solvent. Once the polymers and bioactive agent(s) have been dissolved, the resulting mixture is cast into a die of an appropriate shape.

Then, once cast, the solvent used to dissolve the polymers and bioactive agent(s) is evaporated at a temperature between about 20°C and about 30°C, preferably about 25°C. The polymer can be dried at room temperature or even in a vacuum. For example, the cast polymers including therapeutic bioactive agents can be dried by evaporation in a vacuum.

The dissolving and casting steps form the implants because dissolving the polymers and therapeutic bioactive agents allows the system to naturally partition and form into its most natural configuration based on properties such as polymer viscosity and hence molecular weight, polymer hydrophobicity/hydophilicty, therapeutic bioactive agent molecular weight, therapeutic bioactive agent hydrophobicity/hydophilicty and the like.

Once the cast polymers are dried, they can be processed into an implant using any method known in the art to do so. In one embodiment, the dried casted polymer can be cut into small pieces and extruded into rod shaped structures at a temperature between about 50°C and about 120°C, preferably about 90°C. In other example embodiments, the films can simply be cast without extrusion.

Other methods involve extrusion of dry polymer powders and dry therapeutic bioactive agents. The implants are extruded and formed into a random orientation depending on the dry powder mix itself and not based on physical properties of the components.

The implants may further be formulated with or in a composition with an ophthalmically acceptable suspension, emulsion, or the like. The sustained release formulations described herein can be a high viscosity, polymeric gel to reduce dispersion of one or more implants once placed within an intracameral therapeutic agent delivery device. Preferably, the gel has a high shear characteristic, meaning that the gel can be injected into intracameral therapeutic agent delivery device through a 25-30 gauge needle, and more preferably through a 27-30 gauge needle. A suitable gel for this purpose can be a hydrogel or a colloidal gel formed as a dispersion in water or other aqueous medium. Examples of suitable gels include synthetic polymers such as polyhydroxy ethyl methacrylate, and chemically or physically crosslinked polyvinyl alcohol, polyacrylamide, poly(N-vinyl pyrolidone), polyethylene oxide, and hydrolysed polyacrylonitrile. Examples of suitable hydrogels which are organic polymers include covalent or ionically crosslinked polysaccharide-based hydrogels such as the polyvalent metal salts of alginate, pectin, carboxymethyl cellulose, heparin, hyaluronate and hydrogels from chitin, chitosan, pullulan, gellan, xanthan and hydroxypropylmethylcellulose. Commercially available dermal fillers (such as HYLAFORM^{®} (Biomatrix, Inc., Ridgefiled, NJ), RESTYLANE^{®} (HA North American Sales, Scottsdale, AZ), Sculptura and RADIESSE^{®} (BioForm Medical, Inc., San Mateo, CA)) can be used as the high viscosity gel.

Hyaluronic acid (HA) is a polysaccharide made by various body tissues and can also be used as a high viscosity, polymeric gel to reduce dispersion of one or more implants upon placement of an intracameral therapeutic agent delivery device within an eye. U.S. patent 5,166,331, entitled "Hyaluronics acid fractions, methods for the preparation thereof, and pharmaceutical compositions containing same", discusses purification of different fractions of HA for use as a substitute for intraocular fluids and as a topical ophthalmic therapeutic agent carrier. Other U.S. Patent Applications which discuss ocular uses of HA include U.S. Application Publication No. 20090082321A1, serial number 11/859,627 entitled "Steroid Containing Drug Delivery Systems"; U.S. Application Publication No. 20090149435A1, serial number 11/952,927 entitled "Process For Making A Pharmaceutical Composition"; U.S. Application Publication No. 20050101582A1, serial number 10/966,764 entitled "Compositions and methods for treating a posterior segment of an eye"; U.S. Application Publication No. 20070224278A1, serial number 11/741,366 entitled "Low Immunogenicity Corticosteroid Compositions"; and U.S. Application Publication No. 20050181017A1, serial number 11/039,192 entitled "Compositions and methods for localized therapy of the eye", for all they include regarding ocular uses of HA. The sustained release formulations utilized in accordance with the teachings of the present disclosure preferably comprise a high viscosity HA with an average molecular weight between about 1 and 4 million Daltons, and more preferably with an average molecular weight between about 2 and 3 million Daltons, and most preferably with an average molecular weight of about (± 10%) 2 million Daltons.

Dry uncross-linked HA material comprises fibers or powder of commercially available HA, for example, fibers or powder of sodium hyaluronate (NaHA). The HA may be bacterial-sourced NaHA, animal derived NaHA or a combination thereof. In some embodiments, the dry HA material is a combination of raw materials including HA and at least one other polysaccharide, for example, glycosaminoglycan (GAG).

In some embodiments, the HA compositions comprise or consist of high molecular weight HA. That is, nearly 100% of the HA material in the compositions is a high molecular weight HA. High molecular weight HA means HA with a molecular weight of at least about 1.0 million Daltons (mw ≥ 10⁶ Da) to about 4.0 million Da (mw ≤ 4 x 10⁶ Da). For example, the high molecular weight HA in the present compositions may have a molecular weight of about 2.0 million Da (mw 2 x 10⁶ Da). In another embodiment, the high molecular weight HA may have a molecular weight of about 2.8 million Da (mw 2.8 x 10⁶ Da).

In another embodiment, HA compositions are produced using dry, raw HA material, for example, NaHA, having a desired high/low molecular weight ratio. First, the dry, raw HA material is cleaned and purified. These steps generally involve hydrating the dry HA fibers or powder in the desired high/low molecular weight ratio, for example, using pure water, and filtering the material to remove large foreign matters and/or other impurities. The filtered, hydrated material is then dried and purified. The high and low molecular weight NaHA may be cleaned and purified separately, or may be mixed together, for example, in the desired ratio, just prior to cross-linking.

At this stage in the process, the pure, dried NaHA fibers are hydrated in an alkaline solution to produce an uncross-linked NaHA alkaline gel. Any suitable alkaline solution may be used to hydrate the NaHA in this step, for example, but not limited to an aqueous solution containing NaOH. The resulting alkaline gel will have a pH above 7.5, for example, a pH above 8, for example, a pH above 9, for example, a pH above 10, for example, a pH above 12, for example, a pH above 13.

In one embodiment, the next step in the manufacturing process comprises the step of cross-linking the hydrated, alkaline NaHA gel with a suitable cross-linking agent, for example, butanediol diglycidyl ether (BDDE).

The step of HA cross-linking may be carried out using means known to those of skill in the art. Those skilled in the art appreciate how to optimize the conditions of cross-linking according to the nature of the HA, and how to carry out the cross-linking to an optimized degree. In some embodiments, the degree of cross-linking is at least about 2% to about 20%, for example, is about 4% to about 12%, wherein the degree of cross-linking is defined as the percent weight ratio of the cross-linking agent to HA-monomeric units in the HA composition.

The hydrated cross-linked, HA gel may be neutralized by adding an aqueous solution containing HCl. The gel is then swelled in a phosphate buffered saline solution for a sufficient time and at a low temperature.

In certain example embodiments, the resulting swollen HA gel is a cohesive gel having substantially no visible distinct particles, for example, substantially no visibly distinct particles when viewed with the naked eye. In some embodiments, the gel has substantially no visibly distinct particles under a magnification of less than 35X.

The HA gel is now purified by conventional means for example, dialysis or alcohol precipitation, to recover the cross-linked material, to stabilize the pH of the material and remove any un-reacted cross-linking agent. Additional water or slightly alkaline aqueous solution can be added to bring the concentration of the NaHA in the composition to a desired concentration. In some embodiments, the concentration of NaHA in the composition is in a range between about 10 mg/ml to about 30 mg/ml.

The implants dissolved within a HA composition and associated with a intracameral therapeutic agent delivery device can provide sustained release of the at least one therapeutic bioactive agent one the intracameral therapeutic agent delivery device is properly placed in an eye. In some example embodiments, the HA can delay release of the therapeutic bioactive agent by 3 months, and therefore, controlled release of the therapeutic bioactive agent can be delayed once the intracameral therapeutic agent delivery devices is implanted. In other example embodiments, the HA can help achieve further fine tuning to the controlled release provide by polymers.

The intracameral therapeutic agent delivery devices described herein can be implanted into an eye using an applicator shaped and sized for a particular device. Applicators are known to those having ordinary skill in the art. The devices can be injected from the outside surface of the eye or can be placed within the anterior chamber from within. For example, the intracameral therapeutic agent delivery device illustrated in Figures 2-4 can be injected through the ocular tissue until second end 206 abuts against the outside surface of the ocular tissue it was injected through.

As another example, the intracameral therapeutic agent delivery device illustrated in Figure 8 can be in injected into the ocular tissue from inside the anterior chamber. In other words, an applicator needle including the device is injected trough the ocular tissue into the anterior chamber and then further injected into, for example, the trabecular meshwork. Thereafter, the applicator can be removed completely from the ocular tissue leaving behind the thus implanted intracameral therapeutic agent delivery device.

### Example 1

### Release from Device

A device in accordance with the present disclosure was loaded with sodium fluorescein (NaF) in a polymeric delivery implant. A New Zealand white (NZW) rabbit had this device implanted in the sub-Tenon's space with the inner port extending into the anterior chamber. The Heidelberg retina angiograph (HRA) imaging device demonstrated sustained-release of NaF into the anterior chamber of the rabbit's eye.

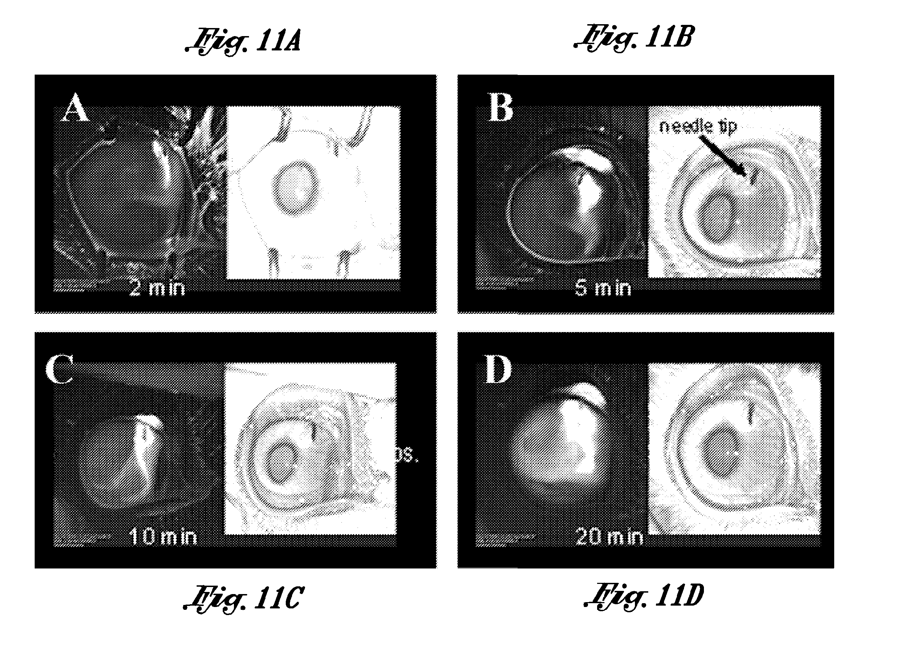

Figure 11 illustrates that in as little as 2 minutes after implantation, the NaF had begun to disperse into the anterior chamber. By 20 minutes, the NaF had substantially dispersed into most of the anterior chamber.

### Example 2

### Treatment of Glaucoma

A 68 year old woman exhibits symptoms of glaucoma including elevated intraocular pressure (IOP). She has previously received topical therapy, but to no avail. Her vision is worsening as time progresses.

The patient receives an intracameral implant as illustrated in Figure 2, the interior of which is coated with a sustained release formulation as described herein including bimatoprost. After 2 weeks, the IOP is reduced.

### Example 3

### Treatment of Macular Degeneration

A 76 year old man has age-related macular degeneration and cataracts in both eyes. The patient also has a history of cardiovascular disease and an inferior wall myocardial infarction 6 months previous. The patient complains of blurry vision and metamorphopsia in the right eye and examination reveals visual acuity of 20/400 right eye, 20/32 left eye. Retinal examination shows subfoveal choroidal neovascularization (CNV) (right eye wet AMD) approximately 1 disc area in size with surrounding hemorrhage and edema in the right eye. The fellow's left eye shows high-risk features for developing wet AMD such as soft, amorphic appearing drusen that included the fovea but no signs of choroidal neovascularization and can be confirmed by fluorescein angiography (left eye dry AMD).

In both eyes, the patient receives an intracameral implant as illustrated in Figure 2, the interior of which was coated with a sustained release formulation as described herein including bimatoprost. At the end of a 7-year follow up period the patient can have maintained vision in the both eyes of at least 20/32

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value or range of values falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification.

Even further, specific embodiments disclosed herein may be further limited in the claims using consisting of or and consisting essentially of language. When used in the claims, whether as filed or added per amendment, the transition term "consisting of" excludes any element, step, or ingredient not specified

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. An intracameral therapeutic agent delivery device (200) comprising:
a substantially cylindrical structure (202) for at least partial placement into the anterior chamber of an eye having a first end (204), a second end (206) and at least one channel (208) traversing the substantially cylindrical structure, said structure formed of at least one biocompatible non-biodegradable material and having at least one protrusion (210) on an outside surface that prevents migration of said device once implanted and wherein one of said first end and said second end is beveled;
a sustained release material associated with at least a portion of said at least one channel; and
at least one therapeutic bioactive agent associated with said sustained release material,
**characterized in that** one of said first end (204) and said second end (206) is sealed with a cap (1006, 1010), and the other of said first end and said second end is sealed with a cap, said caps being pierceable using a syringe (1000).

2. An intracameral therapeutic agent delivery device according to claim 1, wherein said caps comprise silicone.

3. An intracameral therapeutic agent delivery device according to claim 1, wherein said at least one biocompatible non-biodegradable material is a non-biodegradable polymer, a metal, a metal alloy or a combination thereof.

4. An intracameral therapeutic agent delivery device according to claim 1, wherein said sustained release material is a biodegradable polymer or a polymer matrix.

5. An intracameral therapeutic agent delivery device according to claim 1, wherein said sustained release material is packed into said at least one channel as an implant.

6. An intracameral therapeutic agent delivery device according to claim 1, wherein said sustained release material is coated on the inside surface of said at least one channel.

7. An intracameral therapeutic agent delivery device according to claim 1, wherein said therapeutic bioactive agent is a prostaglandin.

8. An intracameral therapeutic agent delivery device according to claim 1, wherein said substantially cylindrical structure comprises a second channel (222).

9. An intracameral therapeutic agent delivery device according to claim 8, wherein said second channel is an aqueous shunt.

10. An intracameral therapeutic agent delivery device according to claim 9, wherein said second channel is an aqueous shunt having sustained release antifibrotic agent.

11. An intracameral therapeutic agent delivery device according to claim 1, wherein said substantially cylindrical structure has at least one therapeutic bioactive agent releasing pore (228) extending from inside said at least one channel to an environment outside said intracameral therapeutic agent delivery device.

## Patentansprüche

1. Intrakamerale Zuführvorrichtung für einen therapeutischen Wirkstoff (200), die aufweist:
eine im Wesentlichen zylindrische Struktur (202) für eine zumindest teilweise Platzierung in einer vorderen Augenkammer, die ein erstes Ende (204), ein zweites Ende (206) und mindestens einen Kanal (208) aufweist, der die im Wesentlichen zylindrische Struktur durchquert, wobei die Struktur aus mindestens einem biokompatiblen nicht abbaubaren Material ausgebildet ist und mindestens einen Vorsprung (210) an einer Außenfläche aufweist, der einer Migration der einmal implantierten Vorrichtung vorbeugt, und wobei das erste Ende oder das zweite Ende angeschrägt ist;
ein Material mit einer verzögerten Freisetzung, das mit zumindest einem Abschnitt des mindestens einen Kanals zusammenhängt; und
mindestens einen therapeutischen bioaktiven Wirkstoff, der mit dem Material mit einer verzögerten Freigabe zusammenhängt,
**dadurch gekennzeichnet, dass** das erste Ende (204) oder das zweite Ende (206) mit einer Kappe (1006, 1010) abgedichtet ist und das jeweils andere des ersten Endes und des zweiten Endes mit einer Kappe abgedichtet ist, wobei die Kappen bei Verwendung einer Spritze (100) durchstechbar sind.

2. Intrakamerale Zuführvorrichtung für einen therapeutischen Wirkstoff nach Anspruch 1, bei der die Kappen Silikon aufweisen.

3. Intrakamerale Zuführvorrichtung für einen therapeutischen Wirkstoff nach Anspruch 1, bei der das mindestens eine biokompatible nicht abbaubare Material ein nicht abbaubares Polymer, ein Metall, eine Metalllegierung oder eine Kombination daraus ist.

4. Intrakamerale Zuführvorrichtung für einen therapeutischen Wirkstoff nach Anspruch 1, bei der das Material mit verzögerter Freigabe ein abbaubares Polymer oder eine Polymermatrix ist.

5. Intrakamerale Zuführvorrichtung für einen therapeutischen Wirkstoff nach Anspruch 1, bei der das Material mit verzögerter Freigabe als Implantat in mindestens einen Kanal gepackt ist.

6. Intrakamerale Zuführvorrichtung für einen therapeutischen Wirkstoff nach Anspruch 1, bei der das Material mit verzögerter Freigabe auf der Innenfläche des mindestens einen Kanals beschichtet ist.

7. Intrakamerale Zuführvorrichtung für einen therapeutischen Wirkstoff nach Anspruch 1, bei welcher der therapeutische bioaktive Wirkstoff Prostaglandin ist.

8. Intrakamerale Zuführvorrichtung für einen therapeutischen Wirkstoff nach Anspruch 1, bei der die im Wesentlichen zylindrische Struktur einen zweiten Kanal (222) aufweist.

9. Intrakamerale Zuführvorrichtung für einen therapeutischen Wirkstoff nach Anspruch 8, bei welcher der zweite Kanal ein Wassershunt ist.

10. Intrakamerale Zuführvorrichtung für einen therapeutischen Wirkstoff nach Anspruch 9, bei welcher der zweite Kanal ein Wassershunt ist, der einen antifibrotischen Wirkstoff mit verzögerter Freigabe aufweist.

11. Intrakamerale Zuführvorrichtung für einen therapeutischen Wirkstoff nach Anspruch 1, bei der die im Wesentlichen zylindrische Struktur mindestens eine Freigabepore für einen therapeutischen bioaktiven Wirkstoff (228) aufweist, die sich von der Innenseite des mindestens einen Kanals zu einer Umgebungsaußenseite der intrakameralen Zuführvorrichtung für einen therapeutischen Wirkstoff erstreckt.

## Revendications

1. Dispositif d'administration d'agent thérapeutique intracamérulaire (200) comprenant :
une structure substantiellement cylindrique (202) pour au moins un positionnement partiel dans la chambre antérieure d'un oeil ayant une première extrémité (204), une deuxième extrémité (206) et au moins un canal (208) traversant la structure substantiellement cylindrique, ladite structure étant formée d'au moins un matériau non-biodégradable biocompatible et ayant au moins une saillie (210) sur une surface extérieure qui empêche le déplacement dudit dispositif une fois implanté, et dans lequel l'une de ladite première extrémité et de ladite deuxième extrémité est biseautée ;
un matériau à libération prolongée associé à au moins une partie dudit au moins un canal ; et
au moins un agent bioactif thérapeutique associé audit matériau à libération prolongée,
**caractérisé en ce que** l'une de ladite première extrémité (204) et de ladite deuxième extrémité (206) est scellée avec un bouchon (1006, 1010), et l'autre de ladite première extrémité et de ladite deuxième extrémité est scellée avec un bouchon, lesdits bouchons pouvant être percés en utilisant une seringue (1000).

2. Dispositif d'administration d'agent thérapeutique intracamérulaire selon la revendication 1, dans lequel lesdits bouchons comprennent de la silicone.

3. Dispositif d'administration d'agent thérapeutique intracamérulaire selon la revendication 1, dans lequel ledit au moins un matériau non-biodégradable biocompatible est un polymère non-biodégradable, un métal, un alliage métallique ou une combinaison de ceux-ci.

4. Dispositif d'administration d'agent thérapeutique intracamérulaire selon la revendication 1, dans lequel ledit matériau à libération prolongée est un polymère biodégradable ou une matrice polymère.

5. Dispositif d'administration d'agent thérapeutique intracamérulaire selon la revendication 1, dans lequel ledit matériau à libération prolongée est implanté dans ledit au moins un canal en tant qu'implant.

6. Dispositif d'administration d'agent thérapeutique intracamérulaire selon la revendication 1, dans lequel ledit matériau à libération prolongée est déposé sur la surface intérieure dudit au moins un canal.

7. Dispositif d'administration d'agent thérapeutique intracamérulaire selon la revendication 1, dans lequel ledit agent bioactif thérapeutique est une prostaglandine.

8. Dispositif d'administration d'agent thérapeutique intracamérulaire selon la revendication 1, dans lequel ladite structure substantiellement cylindrique comprend un deuxième canal (222).

9. Dispositif d'administration d'agent thérapeutique intracamérulaire selon la revendication 8, dans lequel ledit deuxième canal est une dérivation aqueuse.

10. Dispositif d'administration d'agent thérapeutique intracamérulaire selon la revendication 9, dans lequel ledit deuxième canal est une dérivation aqueuse ayant un agent antifibrotique à libération prolongée.

11. Dispositif d'administration d'agent thérapeutique intracamérulaire selon la revendication 1, dans lequel ladite structure substantiellement cylindrique comprend au moins un pore de libération d'agent bioactif thérapeutique (228) s'étendant depuis l'intérieur dudit au moins un canal vers un environnement à l'extérieur dudit dispositif d'administration d'agent thérapeutique intracamérulaire .
